(19) Europäisches Patentamt
European Patent Office
Office européen des brevets

(11) Publication number: 0 107 008 B1

(12) **EUROPEAN PATENT SPECIFICATION**

(45) Date of publication of patent specification: **26.03.86**

(51) Int. Cl.$^4$: **C 07 C 43/166**, C 09 K 11/07

(21) Application number: **83109017.0**

(22) Date of filing: **13.09.83**

(54) **9,10-bis(phenylethynyl)-anthracenes.**

(30) Priority: **25.10.82 US 436211**

(43) Date of publication of application: **02.05.84 Bulletin 84/18**

(45) Publication of the grant of the patent: **26.03.86 Bulletin 86/13**

(84) Designated Contracting States: **AT BE CH DE FR GB IT LI NL SE**

(56) References cited:
**EP-A-0 084 599**
**GB-A-1 425 770**
**US-A-3 557 233**
**US-A-3 597 362**
**US-A-3 729 426**
**US-A-3 749 679**
**US-A-3 911 038**
**US-A-4 226 738**

(73) Proprietor: **AMERICAN CYANAMID COMPANY**
**1937 West Main Street P.O. Box 60**
**Stamford Connecticut 06904 (US)**

(72) Inventor: **Kamhi, Victor Marc**
**110 Herrontown Road**
**Princetown New Jersey 08540 (US)**

(74) Representative: **Wächtershäuser, Günter, Dr.**
**Tal 29**
**D-8000 München 2 (DE)**

**EP 0 107 008 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention, described herein, was made in the performance of work supported by the Office of Naval Research (Contract No. N-00014-77-C-0634), and is subject to the provisions of ASPR 7-104.18, December 1969, and ASPR 7-302.23 (b) long form, August 1977.

The invention relates to novel fluorescer compounds, to mixtures containing those compounds which are useful for generating chemiluminescence, and to the use of such mixtures for generating chemiluminescence.

Zweig and Maulding, in U.S. Patents Nos. 3,557,233 and 3,729,426, and Maulding, in U.S. Patent No. 3,888,786 described 9,1-bis(phenylethynyl)anthracenes substituted by chloro, bromo, fluoro, or lower alkyl groups and their use as fluorescers with certain oxalic-type compounds which react with peroxide to produce chemiluminescence.

Arthen and Manfre, in U.S. Patent Application Serial No. 341,390, filed January 21, 1982, describe 9,10-bis ($C_4$—$C_{22}$ alkylphenylethynyl)anthracenes, mixtures containing such compounds, and the use of such mixtures for generating chemiluminescence.

The invention provides fluorescers for chemiluminescent mixtures that will have higher chemiluminescence efficiencies as measured by the quantum yield, and will have higher light output as measured by the light capacity, than those described by Zweig and Maulding.

The invention provides fluorescers for chemiluminescent mixtures that have slightly lower light capacities and quantum yields than those described by Arthen and Manfre. However, the fluorescers of this invention are more readily soluble in polar organic solvents, such as dimethyl phthalate, diethyl ether, tetrahydrofuran, and ethyl acetate, than the fluorescers of Arthen and Manfre.

Summary of the Invention

The invention provides novel fluorescer compounds having the formula (I),

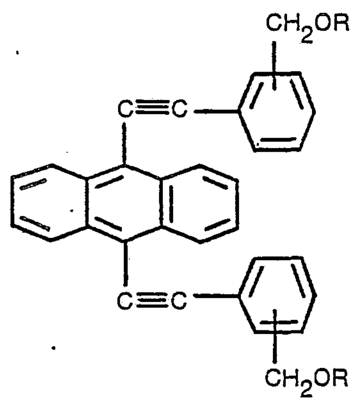

(I)

wherein R is selected from the group consisting of $C_1$ to $C_8$ alkyl, $C_4$ to $C_8$ cycloalkyl, and $C_3$ to $C_6$ alkenyl.

Compounds of formula (I) can be prepared by reacting about two molecular proportions of the corresponding lithium phenylacetylide with anthraquinone to obtain a 9,10-dihydro-9,10-dihydroxy-9,10-bis(phenylethynyl)anthracene and converting the latter to the corresponding 9,10-bis(phenylethynyl)-anthracene by methods described by Maulding in U.S. Patent 3,911,038.

Illustrated examples of suitable compounds of formula (I) include the following:

9,10-bis[2-[4-(methoxymethyl)phenyl]ethynyl]anthracene,
9,10-bis[2-[4-(n-butoxymethyl)phenyl]ethynyl]anthracene,
9,10-bis[2-[3-(n-octyloxymethyl)phenyl]ethynyl]anthracene,
9,10-bis[2-[4-(cyclohexyloxymethyl)phenyl]ethynyl]anthracene,
9,10-bis[2-[4-(allyloxymethyl)phenyl]ethynyl]anthracene,
9,10-bis[2-[4-(methallyloxymethyl)phenyl]ethynyl]anthracene,

The compositions of formula (I) may comprise mixtures of isomers substituted at the 3 and 4 positions of the phenyl moiety.

The term "chemiluminescence," as employed herein, is defined as the generation of electromagnetic radiation between about 300 and 1200 nanometers by means of a chemical reaction.

The term "chemiluminescent reactant" is defined as any compound which enters into a chemical reaction with a peroxide component to produce chemiluminescence. The preferred chemiluminescent reactants for use in this invention are oxalic acid esters, and certain oxamides which are described for example in U.S. Patents Nos. 3,597,362, 3,888,786, and 4,226,738.

The term "composition for reaction with a peroxide component to generate chemiluminescence," as employed herein, is defined as a mixture of a compound of formula (I) and a chemiluminescent reactant in a diluent in concentrations sufficient to produce chemiluminescence by admixing with a peroxide

component. Thus, the initial concentrations of the compound of formula (I), the reactant compound, and the ingredients of the peroxide component in the reaction mixture must be sufficient to produce chemiluminescence.

The term "admixing," as used herein, means "reacting" or sufficiently bringing together component reactants to obtain chemiluminescence.

The fluorescer compound of formula (I) must not readily react with the peroxide component used in this invention, or with the reactant compound, and must be at least partially soluble in the diluent employed.

The composition for reaction with a peroxide component to generate chemiluminescence may comprise a liquid which will solubilize the fluorescer compound of formula (I) and the reactant compound to provide initial concentrations in the reacting system of about $10^{-3}$M to about 10M, preferably about $10^{-1}$M to about 1M, of the reactant compound and about $10^{-5}$M to about $10^{-1}$M, preferably about $10^{-3}$M to $10^{-2}$M, of the fluorescer compound. This liquid diluent must be relatively unreactive toward the other ingredients of the chemiluminescent mixture.

The molar concentrations of the reactant compound, and the fluorescer compound in the composition before addition and reaction with the peroxide component is about 1.1—2.5, preferably about 1.2—1.3, times the molar concentrations of the same materials in the reacting system described above.

Typical diluents which can be used in the composition for reaction with a peroxide component include esters, ethers, aromatic hydrocarbons, chlorinated aliphatic and aromatic hydrocarbons such as those described for the use in U.S. Patents 3,749,679 and 3,888,786. The most preferred diluent is dibutyl phthalate. Solvent combinations may be used but such combinations should not include strongly electron donating solvents, strongly acidic, or strongly basic solvents.

The term "diluent," as used herein, is defined as a liquid solvent, or vehicle, for the compound of formula (I) and the reactant compound.

The term "peroxide component," as used herein, means a solution of a hydrogen peroxide compound, a hydroperoxide compound, or a peroxide compound in a suitable diluent.

The term "hydroperoxide compound" includes (1) hydrogen peroxide and (2) hydrogen peroxide-producing compounds.

Hydrogen peroxide is the preferred hydroperoxide compound and may be employed as a solution of hydrogen peroxide in a solvent or as an anhydrous hydrogen peroxide compound such as sodium perborate, sodium peroxide, and the like. Whenever hydrogen peroxide is contemplated to be employed, any suitable compound may be substituted which will produce hydrogen peroxide.

Diluents which can be employed for the peroxide component include any liquid which is relatively unreactive toward the hydroperoxide, the chemiluminescent reactant, and the fluorescer compound, and which accommodates a solubility to provide at least 0.01M hydroperoxide solution. Solvents suitable as diluents for the hydroperoxide component include water; alcohols, such as diethyl ether, diamyl ether, tetrahydrofuran, dioxane, dibutyldiethyleneglycol, perfluoropropyl ether, and 1,2-di-methoxyethane; and esters, such as ethyl acetate, ethyl benzoate, dimethyl phthalate, dioctylphthalate, propyl formate. Solvent combinations can, of course, be used such as combinations of the above with anisole, tetralin, and chlorobenzene, providing said solvent combination accommodates hydroperoxide solubility. However, strong electron donor solvents such as dimethyl formamide, dimethyl sulfoxide, and hexamethylphosphoramide should not, in general, be used as a major diluent for the peroxide component.

The preferred diluent for the peroxide component is a mixture of about 80 volume percent dimethyl phthalate and about 20 volume percent tertiary butanol.

The hydrogen peroxide concentration in the peroxide component may range from about 0.2M to about 15M. Preferably, the concentration ranges from about 1M to about 2M.

The lifetime and intensity of the chemiluminescent light emitted can be regulated by the use of certain regulators such as:

(1) by the addition of a catalyst which changes the rate of reaction of hydroperoxide with the compound for formula (I). Catalysts which accomplish that objective include those described in M. L. Bender, "Chem. Revs.," Vol. 60, pg. 53 (1960). Also, catalysts which alter the rate of reaction or the rate of chemiluminescence include those accelerators of U.S. Patent No. 3,775,366, and decelerators of U.S. Patent Nos. 3,691,085 and 3,704,231, or

(2) by the variation of hydroperoxide. Both the type and the concentration of hydroperoxide are critical for the purpose of regulation.

Preferably, a weakly basic accelerator, such as sodium salicylate, is included in the peroxide component to control the lifetime of the chemical lighting system. The concentration of weakly basic accelerator used in the peroxide component may range from about $10^{-6}$M to about $10^{-2}$M, preferably from about $10^{-4}$M to about $10^{-3}$M.

The initial concentration of the ingredients of the peroxide component in the reacting system is about 0.15 to 0.60 of the concentrations in the peroxide component since the peroxide component comprises about 15 to about 60 volume percent of the reaction mixture.

The concentration of the hydroperoxide compound in the chemiluminescent reaction is at least equal to the molar concentration of the chemiluminescent reactant and is preferably 1.2 to 5.0 times the concentration of the chemiluminescent reactant in the reacting system described above. The optimum

concentrations must be determined experimentally for each specific system.

The following examples are illustrative of the present invention. All parts are by weight unless otherwise indicated.

Example 1

Preparation of a Mixture of 9,10-bis[2-[3-(and 4-) (allyloxymethyl)phenyl]ethynyl]anthracenes

Lithium amide (1.6 grams; 0.072 mole) and a mixture of 3 (and 4)-(allyloxymethyl)phenylacetylenes (11.4 grams; 0.066 mole) are suspended in dry-dioxane (125 mls) and stirred at reflux temperature, under nitrogen, for 4 hours. The suspension is cooled to 15°C and anthraquinone (6.2 grams; 0.03 mole) is added thereto, along with additional dioxane (50 mls). The resulting mixture is stirred at reflux for 18 hours, and then cooled to room temperature.

Aqueous acetic acid (9 mls; 50%) is added to the reaction mixture, followed by the addition of a solution of stannous chloride dihydrate (20 grams; 0.09 mole) in N,N-dimethylformamide (80 mls). The resulting solution is stirred at room temperature for 4 hours, and then cooled in an ice bath. Glacial acetic acid (50 mls) and dilute sulfuric acid (50 mls; 5N) are added thereto and the reaction mixture is stirred until the yellow-brown precipitate becomes granular. The solid is then collected by filtration, washed with water, and dried under vacuum at room temperature. The dried solid is dissolved in hot methylcyclohexane, and stirred with Magnesol* (Trademark; FMC Corporation), DARCO® Decolorizing Carbon, (ICI Americas, Inc.), and HYFLO® Super-Cel (Johns-Manville Sales Corporation) at ambient temperature for one hour. The mixture is filtered and the filtrate is cooled to obtain a bright yellow crystalline precipitate, which is recovered by filtration and dried under vacuum, m.p. 55—62°C.

Anal. Calculated for $C_{38}H_{30}O_2$: C, 88.03%, H, 5.79%
Found: C, 88.48%, H, 5.89%

Comparison Example 2

Preparation of a Mixture of 9,10-bis[3(and 4)-(hydroxymethyl)phenylethynyl]anthracene

To a stirred solution of the product of Example 1 (5.2 grams; 0.01 mole) in dry dioxane (75 mols) is added selenium (IV) dioxide (2.4 grams; 0.022 mole) and glacial acetic acid (1.8 mls; 0.03 mole), under nitrogen. After stirring at reflux temperature for 3½ hours, analysis by thin layer chromatography (50/50 methylene chloride: ethyl acetate) reveals almost complete absence of the starting material. The hot solution is filtered to remove a black solid, which is washed with hot dioxane (2 × 50 mls). Evaporation of the filtrate and washings yields an orange semi-solid.

Elution of this material through a Waters Prep 500 High Performance Liquid Chromatography (2:1 chloroform/tetrahydrofuran) yields the desired compound as the most polar fraction, m.p. 160—169°C.

Anal. Calculated for $C_{32}H_{22}O_2$: C, 87.67%; H, 5.02%
Found: C, 85.07%; H, 5.55%

The nuclear magnetic resonance and infrared spectra of the product are consistant with the proposed structure.

Examples 3 and 4

Determination of Chemiluminescence

Solutions of 7.5 mls of bis(6-carbopentoxy-2,4,5-trichlorophenyl)oxalate (CPPO) and selected fluorescers (defined in Table I) are made in dibutyl phthalate. Each solution is mixed with 2.5 mls of a peroxide component which consists of hydrogen peroxide and sodium salicylate in 80% dimethyl phthalate-20% (by volume) tertiary butanol. Each of the chemiluminescent reaction mixtures contains initial concentrations of 0.38 M hydrogen peroxide, $1.56 \times 10^{-4}$M sodium salicylate, 0.10M CPPO, and $2.25 \times 10^{-3}$M of the fluorescer. Quantitative measurements of the chemiluminescence of the solutions are carried out by means of a Hirt-Roberts radiometer-spectrophotometer using the procedure described in the Journal of organic Chemistry, Volume 44, page 4115 (1979). The results are shown in Table I. The fluorescer compounds of Examples 1 and 2 provided significantly higher light capacities and quantum yields than the comparison fluorescer (Example 3 which was selected because it illustrates the criticality of the nature of the R radical in formula (I).

TABLE I

| Example | Fluorescer | max(nm) | Light Capacity[a] | Percent Quantum Yield[b] | T75[c] |
|---|---|---|---|---|---|
| 3 | Compound of Example 1 | 515 | 354 | 12.17 | 82 |
| 4 | Comparison Compound of Example 2 | 515 | 246 | 8.61 | 280 |

(a) Lumen hours per liter
(b) Einsteins per mole × 100
(c) Time (in minutes) required for 75% of the total light to be emitted

Example 5

Preparation of a Mixture of 9,10-bis[3 (and 4)-(n-butoxymethyl)phenylethynyl]anthracenes

The procedure of Example 1 is followed substituting a mixture of 3 (and 4)-(n-butoxymethyl)-phenylacetylenes (12.42 grams; 0.066 mole) for the 3 (and 4)-(allyloxymethyl)phenylacetylene to obtain the desired product.

Determination of chemiluminescence in the manner of Examples 4—6 shows significantly higher quantum yield and light capacity than that obtained with the comparison compound of Example 2.

**Claims for the Contracting States: BE CH DE FR GB IT LI NL SE**

1. Compounds of the formula

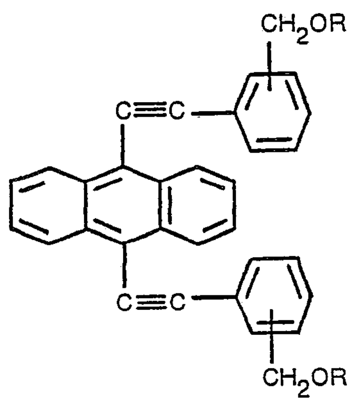

(I)

wherein R is selected from the group consisting of $C_1$ to $C_8$ alkyl, $C_4$—$C_8$ cycloalkyl and $C_3$—$C_6$ alkenyl.

2. A compound according to Claim 1 consisting of 9,10-bis[2-[4-(methoxymethyl)phenyl]ethynyl]-anthracene.

3. A compound according to Claim 1 consisting of 9,10-bis[2-[4-(n-butoxymethyl)phenyl]ethynyl]-anthracene.

4. A compound according to Claim 1 consisting of 9,10-bis[2-[3-(n-octyloxymethyl)phenyl]ethynyl]-anthracene.

5. A compound according to Claim 1 consisting of 9,10-bis[2-[4-(cyclohexyloxymethyl)phenyl]ethynyl]-anthracene.

6. A compound according to Claim 1 consisting of 9,10-bis[2-[4-(allyloxymethyl)phenyl]ethynyl]-anthracene.

7. A compound according to Claim 1 consisting of 9,10-bis[2-[4-(methallyloxymethyl)phenyl]ethynyl]-anthracene.

8. A chemiluminescent mixture comprising a chemiluminescent reactant, a peroxide component and at least one fluorescent compound having the composition defined by Claim 1.

9. A composition for reaction with a peroxide component to generate chemiluminescence comprising at least one fluorescent compound defined by Claim 1.

10. A chemiluminescent mixture defined by Claim 8 wherein the fluorescent compounds consist essentially of 9,10-bis[2-[3- (and 4)-(allyloxymethyl)phenyl]ethynyl]anthracenes.

**Claims for the Contracting State: AT**

1. A chemiluminescent mixture comprising a chemiluminescent reactant, a peroxide component and at least one fluorescent compound of the formula (I)

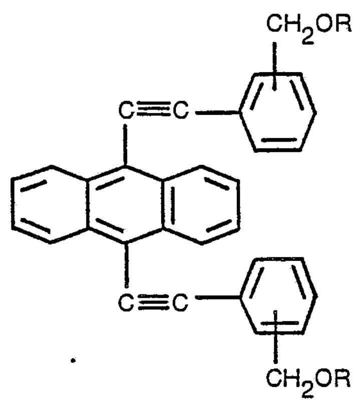

(I)

wherein R is selected from the group consisting of $C_1$ to $C_8$ alkyl, $C_4$—$C_8$ cycloalkyl and $C_3$—$C_6$ alkenyl.

2. A chemiluminescent mixture defined by Claim 1 wherein the fluorescent compounds consist essentially of 9,10-bis[2-[3 (and 4)-(allyloxymethyl)phenyl]ethynyl]anthracenes.

3. A mixture defined by Claim 1, wherein the fluorescent compound is 9,10-bis[2-[4-(methoxymethyl)-phenyl]ethynyl]anthracene.

4. A mixture defined by Claim 1, wherein the fluorescent compound is 9,10-bis[2-[4-(n-butoxymethyl)-phenyl]ethynyl]anthracene.

5. A mixture defined by Claim 1, wherein the fluorescent compound is 9,10-bis[2-[3-(n-octyloxymethyl)-phenyl]ethynyl]anthracene.

6. A mixture defined by Claim 1, wherein the fluorescent compound is 9,10-bis[2-[4-(cyclohexyloxy-methyl)phenyl]ethynyl]anthracene.

7. A mixture defined by Claim 1, wherein the fluorescent compound is 9,10-bis[2-[4-(allyloxymethyl)-phenyl]ethynyl]anthracene.

8. A mixture defined by Claim 1, wherein the fluorescent compound is 9,10-bis[2-[4-(methallyloxy-methyl)phenyl]ethynyl]anthracene.

9. A composition for reaction with a peroxide component to generate chemiluminescence comprising at least one fluorescent compound defined by Claim 1.

**Patentansprüche für die Vertragsstaaten: BE CH DE FR GB IT LI NL SE**

Verbindungen der Formel

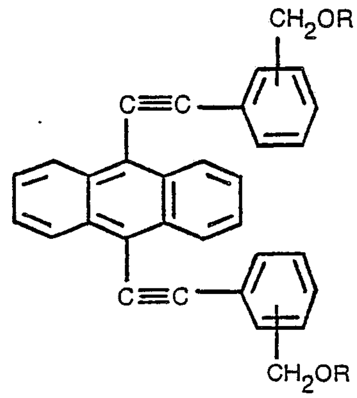

(I)

wobei R ausgewählt ist aus der Gruppe bestehend aus $C_1$—$C_8$-Alkyl, $C_4$—$C_8$-Cycloalkyl und $C_3$—$C_6$-Alkenyl.

2. Verbindung nach Anspruch 1, nämlich 9,10-Bis[2-[4-(methoxymethyl)phenyl]äthinyl]anthracen.

3. Verbindung nach Anspruch 1, nämlich 9,10-Bis[2-[4-(n-butoxymethyl)phenyl]äthinyl]anthracen.

4. Verbindung nach Anspruch 1, nämlich 9,10-Bis[2-[3-(n-octyloxymethyl)phenyl]äthinyl]anthracen.

5. Verbindung nach Anspruch 1, nämlich 9,10-Bis[2-[4-(cyclohexyloxymethyl)phenyl]äthinyl]-anthracen.

6. Verbindung nach Anspruch 1, nämlich 9,10-Bis[2-[4-(allyloxymethyl)phenyl]äthinyl]anthracen.

7. Verbindung nach Anspruch 1, nämlich 9,10-Bis[2-[4-(methallyloxymethyl)phenyl]äthinyl]anthracen.

8. Chemilumineszierendes Gemisch, umfassend einen chemilumineszierenden Reaktanten, eine Peroxidkomponente und mindestens eine Leuchtstoffverbindung mit der in Anspruch 1 angegebenen Zusammensetzung.

9. Eine Zusammensetzung für die Reaktion mit einer Peroxidkomponente zur Erzeugung von Chemilumineszenz, umfassend mindestens eine Leuchtstoffverbindung gemäß Anspruch 1.

10. Chemilumineszierendes Gemisch nach Anspruch 8, wobei die Leuchtstoffverbindungen im wesentlichen aus 9,10-Bis[2-[3 (und 4)-(allyloxymethyl)phenyl]äthinyl]anthracenen bestehen.

**Patentansprüche für den Vertragsstaat: AT**

1. Ein chemilumineszierendes Gemisch, umfassend einen chemilumineszierenden Reaktanten, eine Peroxidkomponente und mindestens eine Leuchtstoffverbindung der Formel (I)

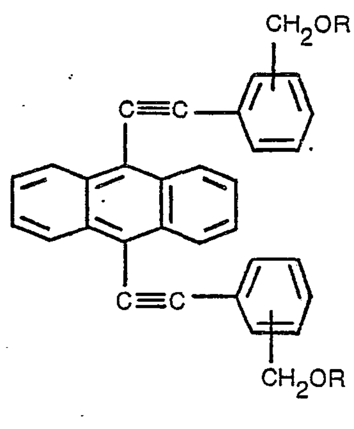

(I)

wobei R ausgewählt ist aus der Gruppe bestehend aus $C_1$—$C_8$-Alkyl, $C_4$—$C_8$-Cycloalkyl und $C_3$—$C_6$-Alkenyl.

2. Ein chemilumineszierendes Gemisch nach Anspruch 1, wobei die Leuchtstoffverbindungen im wesentlichen aus 9,10-Bis[2-[3 (und 4)-(allyloxymethyl)phenyl]äthinyl]anthracenen bestehen.

3. Mischung nach Anspruch 1, wobei die Leuchtstoffverbindung 9,10-Bis[2-[4-(methoxymethyl)phenyl]äthinyl]anthracenen ist.

4. Mischung nach Anspruch 1, wobei die Leuchtstoffverbindung 9,10-Bis[2-[4-(n-butoxymethyl)phenyl]äthinyl]anthracen ist.

5. Mischung nach Anspruch 1, wobei die Leuchtstoffverbindung 9,10-Bis[2-[3-(n-ocytyloxy-methyl)phenyl]äthinyl]anthracen ist.

6. Mischung nach Anspruch 1, wobei die Leuchtstoffverbindung 9,10-Bis[2-[4-(cyclohexyloxymethyl)phenyl]äthinyl]anthracen ist.

7. Mischung nach Anspruch 1, wobei die Leuchtstoffverbindung 9,10-Bis[2-[4-(allyloxymethyl)phenyl]-äthinyl]anthracen ist.

8. Mischung nach Anspruch 1, wobei die Leuchtstoffverbindung 9,10-Bis[2-[4-(methallyloxymethyl)phenyl]äthinyl]anthracen ist.

9. Eine Zusammensetzung für die Reaktion mit einer Peroxidkomponente zur Erzeugung von Chemilumineszenz, umfassend mindestens eine Leuchtstoffverbindung gemäß Anspruch 1.

**Revendications pour les Etats contractants: BE CH DE FR GB IT LI NL SE**

1. Composés de formule:

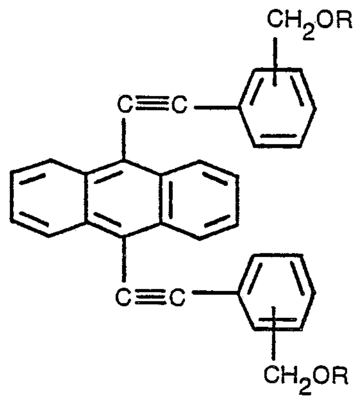

(I)

dans laquelle R est choisi parmi les radicaux alkyle en $C_1$ à $C_8$, cycloalkyle en $C_4$ à $C_8$ et alcényle en $C_3$ à $C_6$.

2. Composé selon la revendication 1 consistant en 9,10-bis[2-[4-(méthoxyméthyl)phényl]éthynyl]-anthracène.

3. Composé selon la revendication 1 consistant en 9,10-bis[2-[4-(n-butoxyméthyl)phényl]éthynyl]-anthracène.

4. Composé selon la revendication 1 consistant en 9,10-bis[2-[3-(n-octyloxyméthyl)phényl]éthynyl]-anthracène.

5. Composé selon la revendication 1 consistant en 9,10-bis[2-[4-(cyclohexyloxyméthyl)phényl]éthynyl]-anthracène.

6. Composé selon la revendication 1 consistant en 9,10-bis[2-[4-(allyloxyméthyl)phényl]éthynyl]-anthracène.

7. Composé selon la revendication 1 consistant en 9,10-bis[2-[4-(méthallyloxyméthyl)phényl]éthynyl]-anthracène.

8. Mélange chimioluminescent comprenant un réactif chimioluminescent, un composant peroxyde et au moins un composé fluorescent défini à la revendication 1.

9. Composition destinée à réagir avec un composant peroxyde pour produire de la chimioluminescence comprenant au moins un composé fluorescent défini à la revendication 1.

10. Mélange chimioluminescent selon la revendication 8 dans lequel les composés fluorescents consistent essentiellement en 9,10-bis[2-[3(et 4)-(allyloxyméthyl)phényl]éthynyl]anthracènes.

**Revendications pour l'Etat contractant: AT**

1. Mélange chimioluminescent comprenant un réactif chimioluminescent, un composant peroxyde et au moins un composé fluorescent de formule:

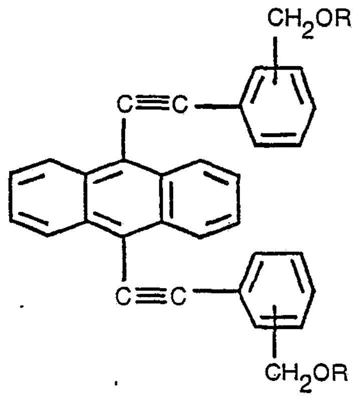

(I)

dans laquelle R est choisi parmi les radicaux alkyle en $C_1$ à $C_8$, cycloalkyle en $C_4$ à $C_8$ et alcényle en $C_3$ à $C_6$.

2. Mélange chimioluminescent selon la revendication 1, dans lequel les composés fluorescents consistent essentiellement en 9,10-bis[2-[3(et 4)-(allyloxyméthyl)phényl]éthynyl]anthracènes.

3. Mélange selon la revendication 1, dans lequel le composé fluorescent est le 9,10-bis[2-[4-(méthoxyméthyl)phényl]éthynyl]anthracène.

4. Mélange selon la revendication 1, dans lequel le composé fluorescent est le 9,10-bis[2-[4-(n-butoxyméthyl)phényl]éthynyl]anthracène.

5. Mélange selon la revendication 1, dans lequel le composé fluorescent est le 9,10-bis[2-[3-(n-octyloxyméthyl)phényl]éthynyl]anthracène.

6. Mélange selon la revendication 1, dans lequel le composé fluorescent est le 9,10-bis[2-[4-(cyclohexyloxyméthyl)phényl]éthynyl]anthracène.

7. Mélange selon la revendication 1, dans lequel le composé fluorescent est le 9,10-bis[2-[4-(allyloxyméthyl)phényl]éthynyl]anthracène.

8. Mélange selon la revendication 1, dans lequel le composé fluorescent est le 9,10-bis[2-[4-(méthallyloxyméthyl)phényl]éthynyl]anthracène.

9. Composition destinée à réagir avec un composant peroxyde pour produire de la chimioluminescence comprenant au moins un composé fluorescent défini à la revendication 1.